# EUROPEAN PATENT APPLICATION

(11) **EP 1 857 145 A1**
(43) Date of publication of application: **21.11.2007**
(21) Application number: 07009711.8
(22) Date of filing: 15.05.2007
(51) Int. Cl.: A61N 5/06, G02B 5/22

(54) **Phototherapy device**

(30) Priority: 19.05.2006 JP 2006140356
(71) Applicant: USHIODENKI KABUSHIKI KAISHA, Chiyoda-ku 100 Tokyo (JP)
(72) Inventor: Sasaki, Masahiro, Tatebayashi-shi, Gunma-ken (JP); Kabeta, Katsutoshi, Himeji-shi, Hyogo-ken (JP)
(74) Representative: Tomerius, Isabel

(57) **Abstract**

Phototherapy device in which, within a housing (1), there is a light source (2), and in which light with given wavelengths emerges from a light exit opening (8) of the housing, at least two filters (12,13) being located between the light source (2) and the light exit opening (8), each of which each absorbs light of different wavelength ranges, the filter (12) on a light incidence side absorbing light with shorter wavelengths than the filter (13) on the light exit side.

## Description

### Background of the Invention

### Field of the Invention

The invention relates to a phototherapy device using a discharge lamp, especially a metal halide lamp, as the light source.

### Background of the Invention

Conventionally, a phototherapy device is known which treats skin disorders and changes tumors into necroses, and thus, treats them using light with a certain wavelength.
As is shown, for example, in Japanese patent JP 3535066 B2, a phototherapy device is known in which the light source is a halogen lamp or a xenon lamp, and in which to irradiate diseased sites with light with wavelengths which are suited for therapy or diagnosis, control is exercised using an interference filter such that light with roughly 400 nm or roughly 500 nm is emitted.

In the control of radiant light using the above described interference filter, there have been the following disadvantages:

In an interference filter, on the surface of the glass substrates several layers with a high index of refraction and several layers with a low index of refraction are superimposed in alternation so that optimum control of the light which is vertically incident in the interference filter can be exercised. However, with respect to the light which is incident obliquely in the interference filter, light with a wavelength true to the structure cannot be reflected. Therefore, there is the potential problem that light with unnecessary wavelengths will be transmitted.

That is, the above described phototherapy device is made such that, within the device, there is a lamp and the light emitted from the lamp is reflected by means of a reflector. Since the light which cannot be captured by the reflector strikes the inner wall within the device, the light which is emitted from the direction outside a given angle is not incident in the interference filter, by which diseased sites are irradiated with light outside the given wavelength. As a result, in the case in which the diseased sites are irradiated with UV radiation outside of given wavelength, there is the danger that the skin will be inflamed by the UV radiation. Furthermore, in the case in which the diseased sites are irradiated with IR radiation outside of given wavelength, there is the danger that the skin will be burned.

Conversely, in the case in which the radiant light is controlled using a light absorption filter, it becomes possible at any angle of the light which is incident in the light absorption filter to reliably absorb specific radiation by the light absorption filter, to transmit only the desired light and to irradiate diseased sites only with light in the desired wavelength range.

### Summary of the Invention

The invention was devised to eliminate the above described disadvantages in the prior art. Thus, a primary object of the present invention is to devise a phototherapy device in which a light absorption filter located between the light source and the light exit opening is prevented from being overly heated, and thus, destruction of the filter by the light incident on the light incidence side as a result of a high temperature is prevented.

According to a first measure, in a phototherapy device in which within the housing there is a light source and in which light with a given wavelength emerges from the light exit opening of the above described housing, the object is achieved in that, between the light source and the light exit opening, there are a filter on the light incidence side and a filter on the light exit side with different light absorption wavelength ranges, with the filter on the light incidence side absorbing light with shorter wavelengths than the filter on the light exit side.

The object is achieved according to a second measure in a phototherapy device according to the first measure in that the filter on the light incidence side is divided essentially through its center.

### Action of the Invention

In a first aspect of the invention, the filter on the light incidence side and the filter on the light exit side absorb light with different wavelengths so that the heat which is formed by light absorption by the respective filter is divided and a temperature increase of the respective filter can be suppressed. Therefore, degradation of the respective filter by heat can be prevented.

In one development, the filter on the light incidence side is located in the housing, this filter being closer to the light source and having a poor heat dissipation state. By dividing the middle region with the highest temperature on the filter, thermal distortion of the filter on the light incidence side can be effectively prevented.

The invention is further described below with reference to the accompanying drawings.

### Brief Description of the Drawings

Figure 1 is a schematic cross section of a phototherapy device in accordance with the invention;

Figure 2 is a graphic representation of the degrees of light transmission with respect to wavelengths of the filter on the light incidence side and of the filter on the light exit side;

Figure 3 is a graph of the spectral distribution of the light emitted from the light source;

Figure 4 is a graph of the spectral distribution of the light emitted from the light exit opening;

Figure 5 is a front view of the filter on the light incidence side which is divided by a line which passes essentially through the center; and

Figure 6 shows a cross section of the filters on the light incidence side and on the light exit side showing that the filter on the light incidence side is divided essentially in the center in a direction which is oblique with respect to the optical axis.

### Detailed Description of the Invention

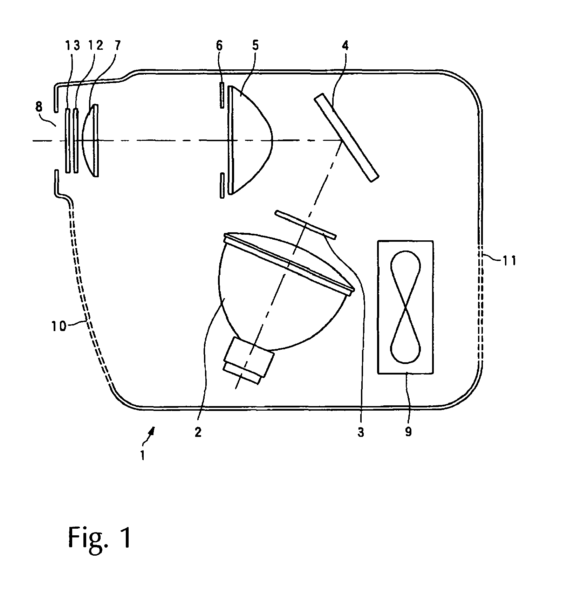

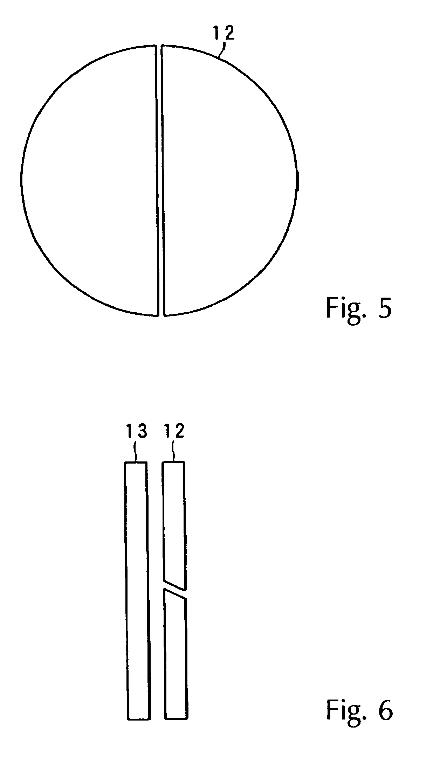

One embodiment of the invention is described below using Figures 1 & 6.

Figure 1 a phototherapy device according to an embodiment of the invention having a housing 1 in which there are a light source 2, a heating wire absorption filter 3 in front of the light source 2 on its light exit side, a reflection mirror 4, a condenser lens 5, an opening 6 in the position in which the distribution of the light intensity is essentially uniform, a projection lens 7 which projects the optical image in the opening 6 onto the surface of the body to be irradiated, enlarged, and a cooling fan 9. The housing has a light exit opening 8, an air intake opening 10 which is provided on the front of the housing 1, and an air outlet opening 11 which is provided on the back side of the housing 1. Moreover, there is a filter pair having a filter 12 on the light incidence side and a filter 13 on the light exit side.

As is shown in Figure 1, in this phototherapy device, the light emerging from the light source 2 is emitted from the light exit opening 8 via the heating wire absorption filter 3, the reflection mirror 4, the condenser lens 5, the opening 6, the projection lens 7, the filter on the light incidence side 12 and the filter on the light exit side 13. In the housing 1, an air intake opening 10 and an air discharge opening 11 are formed. Outside air is supplied to the interior of the housing 1 and inside air is discharged by the action of the cooling fan 9, cooling the interior of the housing 1.

The heating wire absorption filter 3 absorbs light in the IR region of wavelengths of at least 800 nm which is more or less emitted from the light source 2.

The light source 2 is, for example, a metal halide lamp. The inside volume of the arc tube is 0.4 cm³, the rated wattage is 145 W and the lamp voltage is 80 V. The arc tube is filled with 2 mg NaI, 0.2 mg LiI, 0.2 mg HgBr₂, 14 mg mercury and 13.3 kPa Ar as the rare gas. In this metal halide lamp there is little IR radiation of greater than or equal to 740 nm and the radiation intensity of the light with 550 nm to 740 nm is great. In the housing 1, between the light source 2 and the light exit opening 8, is at least one filter 12 on the light incidence side and a filter 13 on the light exit side which absorb light with certain wavelengths.

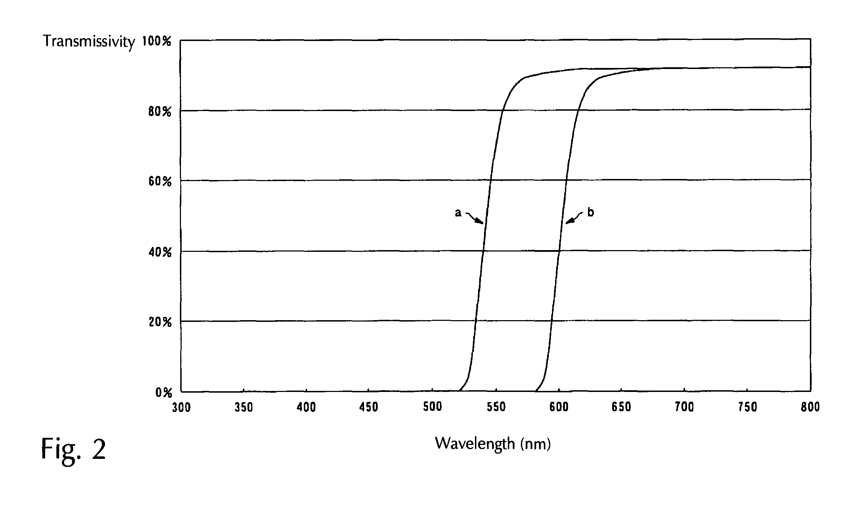

The filter 12 on the light incidence side, i.e., on the side directed toward the light source 2, contains as the main components, besides silicon dioxide, also boron oxide, zinc oxide, selenium, cadmium carbonate and cadmium sulfide. The filter 12 on the light incidence side is made in the form of a disk and has a thickness of 3 mm and a diameter of 44 mm. Its surface was optically polished. This filter 12 on the light incidence side absorbs light with shorter wavelengths than the filter 13 on the light exit side. The degree of light transmission of the filter 12 on the light incidence side has the property which is shown using the graphic representation a in Figure 2. As is apparent from graphic representation a, the filter 12 on the light incidence side absorbs light with wavelengths shorter than 540 nm, i.e., does not transmit it, but transmits light of at least 540 nm.

The filter 13 on the light exit side, i.e., the side facing the light exit opening 8 contains as the main components, besides silicon dioxide, also boron oxide, zinc oxide, selenium, and cadmium sulfide. The filter 13 on the light exit side is made in the form of a disk and has a thickness of 3 mm and a diameter of 44 mm. Its surface is optically polished. The filter 13 on the light exit side absorbs light with wavelengths smaller than 600 nm. The degree of light transmission of the filter 13 on the light exit side has the properties which are shown in the graphic representation b in Figure 2. As is apparent from graphic representation b, the filter 13 on the exit side absorbs light with wavelengths shorter than 600 nm, i.e., does not transmit it, but does transmit light of at least 600 nm.

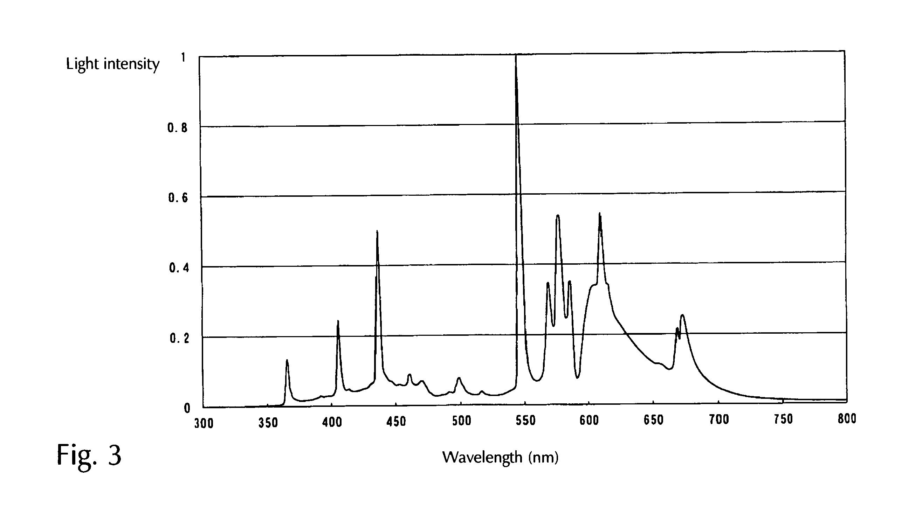

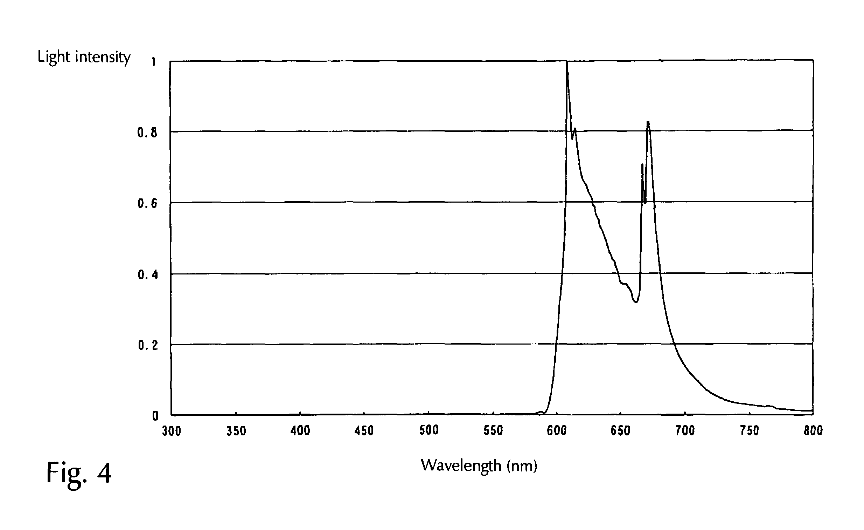

That is, the light emitted by the light source 2 has the spectral distribution shown in Figure 3. Of the light emitted from the light source 2, first of all, light of wavelengths that are shorter than 540 nm is absorbed by the filter 12 on the light incidence side. Furthermore, of the light which has been transmitted by the filter 12 on the light incidence side, light with wavelengths that are shorter than 600 nm is absorbed by the filter 13 on the light exit side so that the light that is emitted from the light exit opening 8 of the housing 1 has wavelengths from 600 nm to 740 nm, as is shown in the spectral distribution as shown in Figure 4.

By the phototherapy device according to this embodiment of the invention, the light emitted from the light source 2 can be controlled by the light absorption filters 12, 13 such that, at any angle of the light which is incident in the light absorption filters 12, 13, light with shorter than 600 nm can be reliably absorbed by the light absorption filters 12, 13, only light of at least 600 nm can be transmitted and diseased sites can be irradiated only with light in the wavelength range of 600 nm to 740 nm.

Furthermore, the filter 12 on the light incidence side absorbs light with shorter than 540 nm The filter 13 on the light exit side in practice absorbs light with 540 nm to 600 nm. Since the respective filters absorb light with different wavelengths, and the heat which has developed can be divided among the two filters, a temperature increase of the respective filter can be suppressed and thermal degradation of the respective filter can be prevented.

Furthermore, there is the danger that the filter 12 on the light incidence side is subject to a great thermal influence and breaks, since it absorbs all light with 540 nm. As is shown in Figure 5, the filter 12 on the light incidence side can be divided beforehand by a line which passes essentially through the middle region of the filter, so that breaking of the filter 12 on the light incidence side can be prevented. The reason why division by the line which passes essentially through the middle region is done beforehand, is that the filter 12 in the housing 1 is located on the light incidence side, that also the heat dissipation state is not good and that by dividing the middle region which forms the region with the highest temperature on the filter, thermal distortion of the filter 12 on the light incidence side can be effectively prevented, since the filter is located in the vicinity of the light source. Instead of dividing the filter 12 on the light incidence side by the line which passes essentially through the center of the filter, division is also possible essentially in the middle region in an oblique direction with respect to the optical axis as shown in Figure 6.

In the phototherapy device according to this embodiment, a case of using two light absorption filters 12, 13 has been described. However, the invention is not limited to two light absorption filters 12, 13, but also at least three light absorption filters proceeding from light absorption filters in the vicinity of the light source to light absorption filters in the vicinity of the light exit opening can be arranged such that the light wavelengths to be absorbed become longer in series. This arrangement of the light absorption filters reduces the heat absorbed by each of the light absorption filters, by which the above described measures of division by the line which passes essentially through the center of the light absorption filter and similar measures need not unconditionally be taken.

## Claims

1. Phototherapy device, comprising:
a housing having an exit opening,
a light source in the housing from which light with given wavelengths emerges from the light exit opening of the housing, and
at least two filters located between the light source and the light exit opening;
wherein said at least two filters comprise a filter on a light incidence side of said at least two filters and a filter on a light exit side of said at least two filters, each of said at least two filters absorbing light of different wavelength ranges, the filter on the light incidence side absorbing light of shorter wavelengths than the filter on the light exit side.

2. Phototherapy device in accordance with claim 1, wherein the filter on the light incidence side has an opening in a middle region.

3. Phototherapy device in accordance with claim 2, wherein said opening in the filter on the light incidence side is a gap which passes essentially through the center of the filter dividing it into two filter halves.

4. Phototherapy device in accordance with claim 3, wherein the gap runs through the filter on the light incidence side in a direction that is oblique with respect to an optical axis in a direction of light propagation through the filter on the light incidence side.
